# EUROPÄISCHE PATENTANMELDUNG

(11) **EP 1 078 905 A1**
(43) Veröffentlichungstag der Anmeldung: **28.02.2001**
(21) Anmeldenummer: 00113883.3
(22) Anmeldetag: 30.06.2000
(51) Int. Cl.: C07C 13/15, C07C 1/24

(54) **Verfahren zur Herstellung von substituierten Cyclopentadienen**

(30) Priorität: 30.07.1999 DE 19935885
(71) Anmelder: TARGOR GmbH, 55116 Mainz (DE)
(72) Erfinder: Bingel, Carsten, Dr., 65830 Kriftel (DE)
(74) Vertreter: Stark, Vera, Dr.

(57) **Zusammenfassung**

Die vorliegende Erfindung betrifft ein Verfahren zur Herstellung von substituierten, insbesonders von 1,3-disubstituierten Cyclopentadienen, die ausgehend von substituierten-Cyclopent-2-en-1-onen hergestellt werden.

## Beschreibung

Die vorliegende Erfindung betrifft ein Verfahren zur Herstellung von substituierten, insbesonders von 1,3-disubstituierten Cyclopentadienen, die ausgehend von substituierten-Cyclopent-2-en-1-onen hergestellt werden.

Alkyl- und arylsubstituierte Cyclopentadiene werden zur Herstellung von Metallocenkomplexen ("Chemistry of Organozirconium and - hafnium compounds -", D. J. Cardin, M. F. Lappert, C. L. Raston; 1986, Ellis Horwood Limited) wie zum Beispiel Metallocenen des Titans, Zirkoniums, Hafniums oder der Lanthanoide eingesetzt. Diese Metallocene sind Bestandteil von hochaktiven Katalysatorsystemen zur Herstellung von Polyolefinen, insbesondere Polyethylenen und Copolymeren von Ethylen mit weiteren α-Olefinen (W09504761).

Die Synthese von substituierten, insbesondere von 1,3-disubstituierten Cyclopentadienen ausgehend von substituierten, insbesondere von in 3-Stellung substituierten Cyclopent-2-en-1-onen ist in der Literatur allgemein beschrieben (EP-A-0729967; EP-A-0648722; Chem. Ber. 109, 329-336 (1976); Tetrahedron (1965), 21,2313) und erfolgt nach Schema (1):

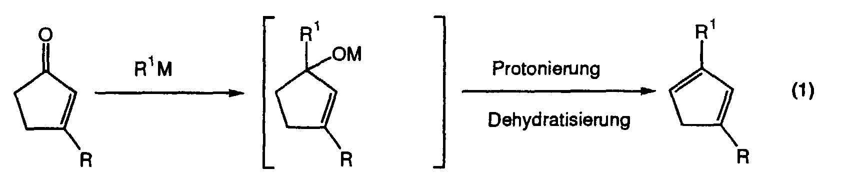

Es bedeuten R, R¹ gleich oder verschieden Alkyl- oder Arylreste und M gleich Li, MgCl, MgBr oder MgI.

Durch die Umsetzung mit einer Grignardverbindung oder Organolithiumverbindung wird ein tertiäres Alkoholat gebildet. Nach Protonierung und mit Säure katalysierter Dehydratisierung entsteht das 1,3-disubstituierte Cyclopentadien. Die nach Schema (1) ablaufende Reaktion findet problemlos in der gezeigten Weise statt, wenn R¹ beispielsweise gleich einem Arylrest oder Tertärbutylrest ist.

Aus EP-A-0648722 ist bekannt, daß bei Verwendung von Alkylgrignards, wie Methyl-, Ethyl- Propyl oder Isopropylmagnesiumhalogenid, nach der Alkoholbildung und der mit Säure katalysierten Dehydratisierung neben den gewünschten Endo-Isomeren (Cyclopentadiene) auch unerwünschte Exo-Isomere entstehen. Dies wird im nachfolgenden Schema (2) beschrieben: 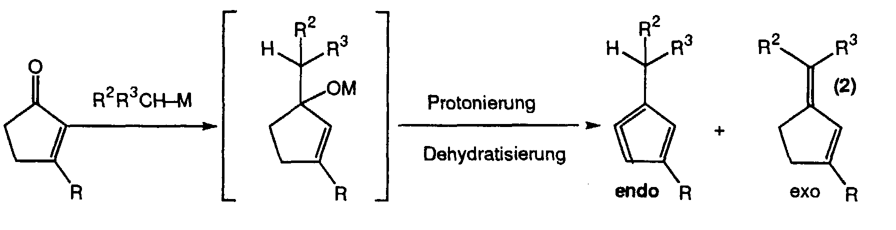 worin M gleich Li, MgCl, MgBr oder MgI und
R, R², R³ gleich oder verschieden Alkyl- oder Arylreste sein können.

Wird - wie in EP-A-0648722 beschrieben - zur Dehydratisierung des tertiären Alkohols eine starke Säure eingesetzt, so erhält man ein Endo / Exo-Isomeren-Verhältnis von etwa 1 : 1. Weiterhin wird in EP-A-0648722 beschrieben, daß bei Verwendung von Carbonsäuren in der Dehydratisierung das Verhältnis von Endo/Exo auf 2.5 : 1 verbessert werden kann.

Nachteilig bei dem in EP-A-0648722 beschriebenen Verfahren ist, daß zunächst die Umsetzung zum Alkoholat in Diethylether / THF durchgeführt wird, und daß anschließend das Alkoholat zu einer wässrigen Carbonsäurelösung gegeben wird. Im technischen Maßstab sollte aus Sicherheitsgründen auf das Lösungsmittel Diethylether verzichtet werden. Weiterhin ist die Verwendung von zwei Reaktoren, einen für die Alkoholatsynthese und einen für die Dehydratisierung, kostspielig.

Es bestand somit die Aufgabe, ein Verfahren zur Herstellung von substituierten, insbesondere von 1,3-disubstituierten Cyclopentadienen zu finden, das die oben genannten Nachteile vermeidet und sich problemlos unter technischen Bedingungen durchführen läßt.

Es wurde nun überraschend gefunden, daß die aus technischer Sicht oben beschriebenen Nachteile trotz Verwendung von starken Mineralsäuren zur Dehydratisierung durch eine geeignete Reaktionsführung vermieden werden können, ohne die Endo/Exo-Selektivität zu verschlechtern.

Die vorliegende Erfindung betrifft somit ein Verfahren zur Herstellung von substituierten, insbesondere von 1,3-disubstituierten Cyclopentadienen umfassend die Schritte:
a) Umsetzung einer Verbindung der Formel (I) 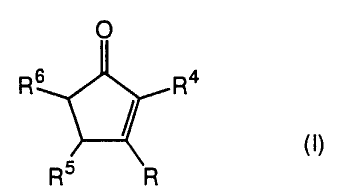 mit einer Verbindung der Formel (II)

   R²R³CH-M (II)

   worin
   - R: gleich ein C₁-C₂₀ Kohlenwasserstoffrest, bevorzugt gleich Alkyl oder Aryl und besonders bevorzugt gleich Methyl oder Phenyl ist, und
   - R², R³: gleich oder verschieden Wasserstoff oder ein C₁-C₂₀ Kohlenwasserstoffrest, bevorzugt Wasserstoff, Alkyl oder Aryl, besonders bevorzugt Wasserstoff oder ein linearer oder verzweigter C₁-C₈ Alkylrest ist, und
   - R⁴, R⁵, R⁶: gleich oder verschieden Wasserstoff oder ein C₁-C₂₀ Kohlenwasserstoffrest, bevorzugt Wasserstoff, Alkyl oder Aryl, besonders bevorzugt Wasserstoff ist, und
   - M: gleich Li, MgCl, MgBr oder MgI bedeuten
   in einem oder mehreren inerten organischen Lösungsmittel zu einer Verbindung der Formel (III) 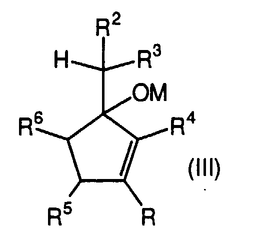
b) gegebenenfalls Abtrennen des inerten organischen Lösungsmittels aus Schritt a) und Zugabe von einem oder mehreren mit Wasser nicht mischbaren organischen Lösemitteln
c) Zugabe einer starken, wässrigen anorganischen Säure, um eine Protonierung und Dehydratisierung der in Schritt a) gebildeten Verbindung der Formel (III) unter Ausbildung der Verbindungen der Formel (IVa) und (IVb) zu erreichen. 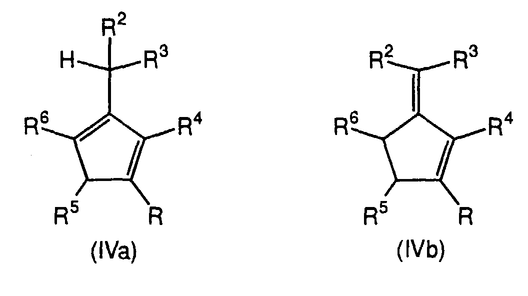
d) Abtrennen der sauren wäßrigen Phase und Aufarbeitung der die Verbindungen der Formel (IVa) und (IVb) enthaltenden organischen Phase.

Bei dem erfindungsgemäßen Verfahren wird zunächst ein substituiertes, insbesondere ein in 3-Stellung substituiertes-Cyclopent-2-en-1-on mit einer Grignardverbindung oder einer Organolithiumverbindung MCHR²R³ zum tertiären Alkoholat umgesetzt. Anschließend wird im gleichen Reaktor durch Zugabe einer starken anorganischen Säure das Alkoholat in einem wässrigen Zweiphasensystem zu einem Endo/Exo-Isomerengemisch dehydratisiert wird, wobei der erwünschte Endo-Anteil mindestens 70 % (bezogen auf Gesamtmenge Endo/Exo) beträgt.

Die organische Lösungsmittelphase darf mit der wässrigen sauren Phase praktisch nicht mischbar sein, um eine mit Säure katalysierte Isomerisierung vom Endo-Isomer zum Exo-Isomer zu vermeiden. Auch die Anwesenheit von sauren Verbindungen, die sowohl in der wässrigen als auch der organischen Phase löslich sind, ist zu vermeiden. Enthält die organische Phase größere Anteile eines mit Wasser mischbaren Lösungsmittels, so verschlechtert sich das Endo/Exo-Verhältnis. Dieses Problem konnte durch einen partiellen Lösungsmittelwechsel gelöst werden, indem beispielsweise ein mit Wasser mischbares Lösungsmittel wie Tetrahydrofuran teilweise abdestilliert wird, durch einen Kohlenwasserstoff, wie ein Alkan oder einen Aromaten oder entsprechende Gemische, beispielsweise Pentan, Hexan, Isohexan, Heptan, ®Exxsol DSP 100 - 120, Toluol, Xylol, Ethylbenzol, Tetrahydronaphthalin, vorzugsweise Heptan, ersetzt wird und anschließend erst die wässrige Mineralsäure zugegeben wird. Die Reaktion läuft nach folgendem Schema ab:

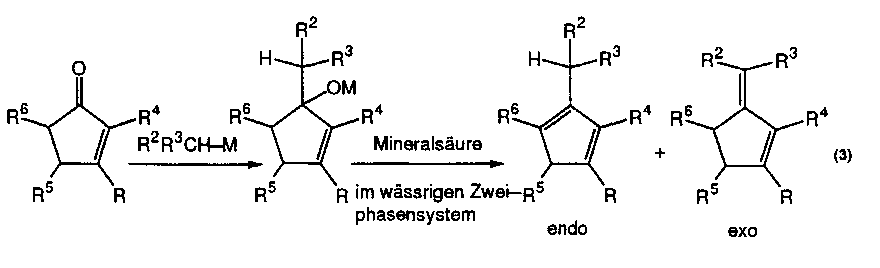

Als starke, wässrige anorganische Säuren werden vorzugsweise solche eingesetzt, deren pKs-Wert kleiner als 4 ist. Insbesondere wird Salzsäure, Schwefelsäure oder Phosphorsäure eingesetzt.

Die substituierten-2-Cyclopenten-1-one sind zum Teil literaturbekannt. Die bevorzugten in 3-Stellung substituierten-2-Cyclopenten-1-one lassen sich nach literaturbekannten Verfahren beispielsweise aus 1,4-Diketonen herstellen (J. Chem. Soc. 1127 (1952); US 5,026,919; J. Org. Chem. 55, 311-315 (1990); Tetrahedron 39, 4127-4132 (1983)). Bevorzugt werden 3-Methyl- oder 3-Phenyl-2-cyclopenten-1-one.

Die metallorganischen Verbindungen R²R³CHM, wobei R², R³ und M wie oben definiert sind, sind kommerziell erhältlich oder lassen sich nach literaturbekannten Verfahren leicht herstellen ( zum Beispiel Grignardverbindungen: Organikum, 15. Auflage, VEB Deutscher Verlag der Wissenschaften, Berlin 1981, S 623).

Die Umsetzung der metallorganischen Verbindung mit dem Cyclopent-2-en-1-on kann im Verhältnis 1 : 1 bis 1.5 : 1, vorzugsweise 1.1 bis 1.3 : 1 in etherhaltigen Lösungsmitteln oder Lösungsmittelgemischen, die einen Ether, jedoch nicht Diethylether, und einen Kohlenwasserstoff enthalten, vorzugsweise Tetrahydrofuran als Ether und Heptan oder Toluol als Kohlenwasserstoff, in einem Temperaturbereich von -78°C bis 110°C, bevorzugt - 10°C bis 50°C durchgeführt werden.

Nach der Zusammengabe der beiden Reaktionskomponenten wird noch 0 bis 5 h, vorzugsweise 1 bis 3 h bei einer Temperatur von 0°C bis 110°C, vorzugsweise 20°C bis 50°C, nachgerührt, bevor bei vermindertem Druck, 800 mbar bis 100 mbar, bevorzugt 400 bis 600 mbar der Ether, insbesondere Tetrahydrofuran, weitestgehend abdestilliert wird.

Das abdestillierte mit Wasser mischbare Lösungsmittel bzw. der Lösungsmittel-bestandteil wird durch einen mit Wasser nicht mischbaren Kohlenwasserstoff, wie oben beschrieben, vorzugsweise n-Heptan, ersetzt.

Zu der Suspension des Alkoholates im Kohlenwasserstoff wird bei einer Temperatur von -30°C bis 50°C, vorzugsweise -5°C bis 10°C, unter kräftigem Rühren verdünnte Mineralsäure, vorzugsweise Salzsäure, Schwefelsäure oder Phosphorsäure, besonders bevorzugt verdünnte Schwefelsäure zugetropft, wobei das Volumen der wässrigen sauren Phase zum Volumen der organischen Phase 2 : 1 bis 0.5 : 1 beträgt. Das molare Verhältnis der Protonen zur eingesetzten metallorganischen Verbindung beträgt 1 : 1 bis 4 : 1, bevorzugt 1.5 : 1 bis 2.5 : 1. Der pH-Wert der wässrigen Phase ist nach der vollständigen Säurezugabe kleiner als 4, bevorzugt kleiner als 2.

Nach Zugabe der Säure wird noch bis zu 30 min nachgerührt, bis die Dehydratisierung des tertiären Alkohols vollständig abgeschlossen ist.

Die gesamte Reaktionsführung zeichnet sich dadurch aus, daß die verschiedenen Reaktionsschritte, die eventuelle Herstellung einer metallorganischen Verbindung, zum Beispiel die Herstellung einer Grignard-Verbindung, die Umsetzung des substituierten Cyclopent-2-en-1-ons mit der metallorganischen Verbindung, die anschließende Protonierung des tertiären Alkoholates und die mit Säure katalysierte Dehydratisierung des tertiären Alkohols zum substituierten Cyclopentadien, alle hintereinander im selben Reaktionsgefäß durchgeführt werden.

Bei der Dehydratisierung entsteht neben dem gewünschten Endo-Isomer, dem Cyclopentadienderivat, auch das unerwünschte Exo-Isomer, wobei das Verhältnis Endo/Exo > 1.5 : 1, bevorzugt > 2 : 1 ist.

Das Endo/Exo-Isomerengemisch kann durch Destillation unter vermindertem Druck erhalten werden. Der Gehalt an dem Endo-Isomer kann mittels Gaschromatographie bestimmt werden, um die zur Deprotonierung des substituierten Cyclopentadiens benötigte Menge Base, wie zum Beispiel Butyllithium, Methyllithium, Phenyllithium, Dibutylmagnesium, Butylmagnesiumchlorid, Natriumhydrid oder Kaliumhydrid, zu berechnen. Das so hergestellte substibuierte-Cyclopentadienyl-Alkali- oder - Erdalkali-salz kann nach bekannten Methoden durch Umsetzung mit den Halogeniden von Titan, Zirkonium, Hafnium oder der Lanthanoide, bevorzugt Zirkoniumtetrachlorid oder den Etherkomplexen von ZrCl₄, wie zum Beispiel ZrCl₄*2THF oder ZrCl₄*DME, zu den entsprechenden Metallocen umgesetzt werden.

Die Erfindung wird durch folgende Beispiele, die jedoch keine Beschränkung der Erfindung darstellen sollen, illustriert.

### Beispiel 1a)

### Darstellung von 1,3-n-Butyl-methyl-cyclopentadien

Zu 41 ml (110 mmol) einer Lösung von Butyllithium in Toluol (2.68 molar) wurde eine Lösung von 9.9 ml (100 mmol) 3-Methyl-2-cyclopent-2-en-1-on in 16 ml Toluol / 4 ml THF bei -10°C bis 30°C zugetropft. Es wurde 1 Stunde bei 30°C nachgerührt. Anschließend wurden 30 ml Toluol zugegeben. Bei 0°C bis 5°C wurden zunächst 10 g Wasser und dann 36 ml 2 molare Schwefelsäure unter kräftigem Rühren zugetropft. Nach 0.5 h wurden die Phasen getrennt, die organische Phase mit 20 ml 2 molarer Schwefelsäure, 40 ml gesättigter Natriumhydrogencarbonat und 40 ml gesättigter Kochsalzlösung gewaschen und mit 5 g Magnesiumsulfat getrocknet. Das GC des Rohproduktes zeigt ein Endo/Exo-Verhältnis von 2.4 : 1.

### Beispiel 1b)

### Darstellung von 1,3-n-Butyl-methyl-cyclopentadien

Die Darstellung von n-Butylmagnesiumchlorid und die anschließende Addition an 3-Methyl-2-cyclopent-2-en-1-on wurde in einer trockenen mit Schutzgas gespülten Apparatur unter Schutzgas durchgeführt.

34.6 g (1.42 mol) Magnesium wurden in 60 ml THF vorgelegt und mit 1.0 ml Dibromethan versetzt. 115.5 g (1.25 mol) 1-Chlor-butan wurden als Lösung in 250 ml THF zu den Magnesiumspänen getropft, wobei die Zutropfgeschwindigkeit so eingestellt wurde, daß das Reaktionsgemisch leicht am Rückfluß siedete. Nach beendeter Zugabe wurde die Grignardlösung noch 2 h bei 75°C nachgerührt. Die Grignardlösung wurde auf 0°C abgekühlt und eine Lösung von 104.3 g (1.06 mol) 3-Methyl-2-cyclopent-2-en-1-on in 60 ml THF und 205 ml n-Heptan wurde so zugetropft, daß die Innentemperatur 30°C nicht überschritt. Anschließend wurde die Suspension 2 h bei 30°C nachgerührt. Bei 500 mbar wurde der größte Teil des THF zusammen mit Heptan abdestilliert (Badtemperatur: 70°C; Kopftemperatur: 41°C; 260 ml Lösungsmittel). Anschließend wurden zu der breiartigen Suspension 380 ml n-Heptan gegeben und der Kolbeninhalt in einem Kochsalz-Eis- Gemisch auf 0°C abgekühlt. Unter Rühren wurden zunächst langsam insgesamt 620 ml 2 molare-Schwefelsäure so zudosiert, daß die Temperatur 5°C nicht überschritt. Nach beendeter Säurezugabe wurde noch 10 Minuten nachgerührt. Im Scheidetrichter wurde das viskose Zweiphasengemisch getrennt, die wässrige saure Phase noch 2 mal mit insgesamt 260 ml n-Heptan extrahiert. Die vereinigten organischen Phasen wurden mit 150 ml Natriumhydrogencarbonatlösung (gesättigt) und mit 150 ml Natriumchloridlösung (gesättigt) gewaschen und mit 40 g Magnesiumsulfat getrocknet (30 Minuten). Nach dem Entfernen des Lösungsmittels am Rotationsverdampfer (Bad: 40°C; 50 mbar) wurden 173 g Rohprodukt erhalten. Das Rohprodukt wurde im Ölpumpenvakuum über eine 30 cm Vigreux-Kolonne mit Kolonnenkopf destilliert (Bad: 83-102°C; Kopf: 52-48°C; 8.5-7.2 mbar). Es wurden 100.4 g einer leicht gelblichen Flüssigkeit erhalten, die laut GC die gewünschten 1,3-Butyl-methyl-cyclopentadien-Isomere zu den Exo-Isomeren im Verhältnis 2.8 : 1 enthielten.

### Vergleichsbeispiel 2a)

### Darstellung von 1,3-n-Butyl-methyl-cyclopentadien in Gegenwart von HNEt₃Cl

Zu 42 ml (120 mmol) einer Lösung von Butylmagnesiumchlorid in THF wurde eine Lösung von 9.9 ml (100 mmol) 3-Methyl-2-cyclopent-2-en-1-on in 20 ml Heptan / 6 ml THF bei 20°C bis 40°C zugetropft. Es wurde 1 Stunde bei 30°C nachgerührt. Anschließend wurden 29 ml Heptan zugegeben. Bei 0°C bis 5°C wurden zunächst 3 g Wasser, 1.4 ml (10 mmol) Triethylamin und dann 31 ml 2 molare Schwefelsäure unter kräftigem Rühren zugetropft. Nach 0.2 h wurden die Phasen getrennt, die organische Phase mit 40 ml gesättigter Natriumhydrogencarbonat und 40 ml gesättigter Kochsalziösung gewaschen und mit 5 g Magnesiumsulfat getrocknet. Das GC des Rohproduktes zeigt ein Endo/Exo-Verhältnis von 0.9 : 1.

### Vergleichsbeispiel 2b)

### Darstellung von 1,3-n-Butyl-methyl-cyclopentadien in THF/Hexan. 1.8 : 1

3.16 g (0.13 mol) Magnesium wurden in 6.5 ml THF vorgelegt und mit 0.2 ml Dibromethan versetzt. 10.2 g (0.11 mol) 1-Chlor-butan wurden als Lösung in 25 ml THF zu den Magnesiumspänen getropft, wobei die Zutropfgeschwindigkeit so eingestellt wurde, daß das Reaktionsgemisch leicht am Rückfluß siedete. Nach beendeter Zugabe wurde die Grignardlösung noch 2 h am Rückfluß erhitzt. Die Grignardlösung wurde auf 0°C abgekühlt und eine Lösung 9.9 ml (100 mmol) 3-Methyl-2-cyclopent-2-en-1-on in 20 ml Heptan / 6 ml THF zugetropft. Es wurde 1 Stunde bei 30°C nachgerührt. Bei 0°C bis 25°C wurden zunächst 10 g Wasser und dann 40 ml 2 molare Schwefelsäure unter kräftigem Rühren zugetropft. Nach 0.1 h wurden die Phasen getrennt, die organische Phase mit 40 ml Wasser gewaschen und mit 5 g Magnesiumsulfat getrocknet. Das GC des Rohproduktes zeigt ein Endo/Exo-Verhältnis von 1 : 1.

### Vergleichsbeispiel 2c)

### Darstellung von 1,3-n-Butyl-methyl-cyclopentadien in THF/Toluol 1 : 1.6

3.16 g (0.13 mol) Magnesium wurden in 6.5 ml THF vorgelegt und mit 0.2 ml Dibromethan versetzt. 11.1 g (0.12 mol) 1-Chlor-butan wurden als Lösung in 25 ml THF zu den Magnesiumspänen getropft, wobei die Zutropfgeschwindigkeit so eingestellt wurde, daß das Reaktionsgemisch leicht am Rückfluß siedete. Nach beendeter Zugabe wurde die Grignardlösung noch 2 h am Rückfluß erhitzt. Die Grignardlösung wurde auf -10°C abgekühlt und eine Lösung 9.9 ml (100 mmol) 3-Methyl-2-cyclopent-2-en-1-on in 20 ml Toluol zugetropft. Es wurde 2 Stunden bei 24°C nachgerührt. Es wurden 30 ml Toluol zugegeben. Bei 0°C bis 25°C wurden zunächst 3 g Wasser und dann 60 ml 2 molare Schwefelsäure unter kräftigem Rühren zugetropft, wobei die Temperatur bis auf 40°C stieg. Anschließend wurde noch 1 h bei 60°C nachgerührt. Die Phasen wurden getrennt, die organische Phase mit 40 ml Wasser gewaschen und mit 5 g Magnesiumsulfat getrocknet. Das GC des Rohproduktes zeigt ein Endo/Exo-Verhältnis von 1 : 1.4.

## Patentansprüche

1. Verfahren zur Herstellung von substituierten Cyclopentadienen umfassend die Schritte:
a) Umsetzung einer Verbindung der Formel (I) 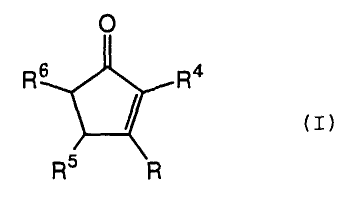 mit einer Verbindung der Formel (II)
R²R³CH-M (II)
worin
R gleich ein C₁-C₂₀ Kohlenwasserstoffrest ist, und
R², R³ gleich oder verschieden Wasserstoff oder ein C₁-C₂₀ Kohlenwasserstoffrest, bevorzugt Wasserstoff, Alkyl oder Aryl, besonders bevorzugt Wasserstoff oder ein linearer oder verzweigter C₁-C₈ Alkylrest ist, und
R⁴, R⁵, R⁶ gleich oder verschieden Wasserstoff oder ein C₁-C₂₀ Kohlenwasserstoffrest, bevorzugt Wasserstoff, Alkyl oder Aryl, besonders bevorzugt Wasserstoff ist, und
M gleich Li, MgCl, MgBr oder MgI bedeuten
in einem oder mehreren inerten organischen Lösungsmittel zu einer Verbindung der Formel (III) 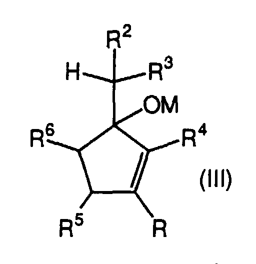
b) gegebenenfalls Abtrennen des inerten organischen Lösungsmittels aus Schritt a) und Zugabe von einem oder mehreren mit Wasser nicht mischbaren organischen Lösemitteln
c) Zugabe einer starken, wässrigen anorganischen Säure, um eine Protonierung und Dehydratisierung der in Schritt a) gebildeten Verbindung der Formel (III) unter Ausbildung der Verbindungen der Formel (IVa) und (IVb) zu erreichen. 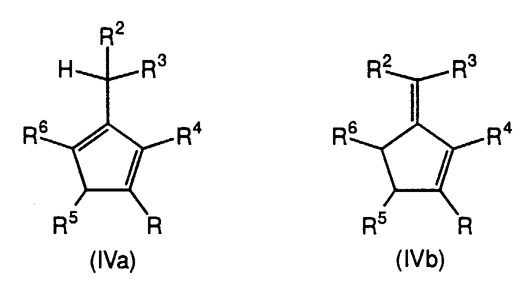
d) Abtrennen der sauren wäßrigen Phase und Aufarbeitung der die Verbindungen der Formel (IVa) und (IVb) enthaltenden organischen Phase.

2. Verfahren gemäß Anspruch 1, dadurch gekennzeichnet, daß als starke, wässrige anorganische Säure eine Säure mit einem pKs-Wert kleiner 4 eingesetzt wird.

3. Verfahren gemäß Anspruch 2, dadurch gekennzeichnet, daß Salzsäure, Schwefelsäure oder Phosphorsäure eingesetzt wird.

4. Verfahren gemäß einem der Ansprüche 1 bis 3, dadurch gekennzeichnet, daß in Schritt a) ein oder mehrere mit Wasser nicht mischbare inerte organische Lösungsmittel eingesetzt werden und Schritt b) entfallen kann.
